# EUROPEAN PATENT SPECIFICATION

(11) **EP 0 720 468 B1**
(45) Date of publication and mention of the grant of the patent: **05.03.1997**
(21) Application number: 94927722.2
(22) Date of filing: 27.09.1994
(51) Int. Cl.: A61J 1/00

(54) **FREEZING FRAMES FOR BLOOD BAGS**
GEFRIERRAHMEN FÜR BLUTBEUTEL
CADRES DE CONGELATION POUR SACS DE SANG

(30) Priority: 02.10.1993 GB 9320373
(43) Date of publication of application: 10.07.1996
(73) Proprietor: SECRETARY OF STATE FOR DEFENCE IN HER BRITANNIC MAJESTY'S GOV. OF THE UNITED KINGDOM OF GREAT BRITAIN AND NORTHERN IRELAND, London SW1A 2HB (GB)
(72) Inventor: BELL, Susan, Helen, Southampton, Hampshire S01 5FN (GB)
(74) Representative: Skelton, Stephen Richard
(86) International application number: GB9402094
(87) International publication number: WO9509597

(56) References cited:
- WO-A-90/09184
- US-A- 4 090 374
- US-A- 4 469 227

## Description

The present invention relates to frames for constraining bags holding blood cells which are to be preserved by freezing for future transfusion.

Blood used for transfusion is normally stored in refrigerators, at a temperature of 4°⁺ ₋ 2°, where its safe life is no longer than six weeks. Potentially longer storage life might be obtained by freezing, but unfortunately freezing blood as obtained from a donor results in destruction of the red blood cells making the blood totally unsuitable for transfusion. Red blood cells can be stored after separation from other blood components, usually by centrifuging, from the associated plasma, mixing with a cryoprotectant, and then freezing. The usual cryoprotectant is glycerol. As glycerol itself is poisonous frozen cells must have all the glycerol removed therefrom before being used for transfusion. The necessary removal process, involving centrifugation and multiple washing steps, is a skilled and time-consuming task which also results in a significant loss of viable blood cells.

Another cryoprotectant is hydroxyethyl starch (HES), which is a widely used artificial plasma expander and is non-toxic. Much work has been carried out on methods of preserving red blood cells using this substance. Early efforts were unsuccessful, as it was found that the level of haemolysis (breakdown of red blood cells) in units of blood (a unit of blood being the volume of a standard donation, about 450ml) recovered after freezing was above the safe limit. It is usually considered that a unit of blood is safe for transfusion if the level of haemolysis at thaw is no greater than 1%. A method of preserving and recovering red blood corpuscules by freezing, using HES as a cryoprotectant, wherein the level of haemolysis after recovery is within acceptable limits, has now been developed and is described in Patent Application PCT/GB90/00140.

The bags used in the method of PCT/GB90/00140 must be capable of fulfilling stringent criteria. Freezing is carried out in liquid nitrogen, and is accompanied by unavoidable changes in volume of the mixture of red blood cells and cryoprotectant. As explained in PCT/GB90/00140 the manner in which this change of volume is accommodated by control of the thickness of the freezing bag during freezing is critical. It is also important that the concentration of HES is within stringent limits, and that the process of transferring donated blood to the freezing bag is carried out in a sterile manner.

Also it is important that the bags, which must, once filled with blood, be stored at sub-zero temperatures of, for example, -100°C, should be of a convenient shape for storage. It must also be possible to allow the blood, after thawing to flow from the bag in a sterile manner for transfusion purposes. Bags which fulfil these criteria are described in Patent Applications PCT/GB88/00947 and PCT/US91/00192. The manner of extraction from these involves penetration by a needle of a special port built into the bag. This procedure requires skill, presents the blood in a format not usually recognised by nursing staff, and also breaches the absolute microbiological integrity of the bag.

A freezing bag which fulfils the requirements for freezing blood according to the method of PCT/GB90/00140, which complies with the required microbiological standards and whose use, once the contents have been prepared for transfusion, is similar to bags as used for conventional transfusions, is described in our Application GB-A-9218538.

It is well known in the art that freezing bags containing red blood cells to be frozen using HES as a cryoprotectant must be constrained within frames, known as freezing frames, to maintain the thickness of the contents substantially constant over the total volume during the freezing process. As is explained in PCT/GB90/00140 small local variations in thickness can have, in practice, a significant effect on overall haemolysis, and as also explained therein the use of freezing frames having cylindrically or, preferably, spherically curved plates can help to avoid such local variations. The manufacture of spherically curved plates for freezing frames is technically demanding, and furthermore the spherically curved shape of bags frozen in such frames is inconvenient for storage purposes. In consequence cylindrically curved frames are preferred and it has been found that using these with the bags according to GB-A-9218538, when the frames are suspended with their lengths vertical (in practice the most convenient orientation for the freezing process), there is a tendency for even the plates of these frames to distort at the lower end due to the effects of gravity added to the freezing effects.

According to the present invention a freezing frame having two cylindrically curved perforated plates adapted to, in use, contain a freezing bag during a freezing process in which the bag is suspended with its length vertical in a freezing medium, has, on each plate, a series of horizontally disposed ribs extending into the volume of the frame.

The ribs in the two plates will usually be staggered.

The freezing bag described in GB-A-9218538 has, in a preferred embodiment, a tube positioned on a port and lying within a pouch which is formed by extensions of the two sheets of material which are bonded together to form a containment zone, the open ends of the pouch being bonded together after transfer of contents to the containment zone and prior to freezing. A freezing frame according to the invention for use with such a bag is therefore sized to contain the entire bag and has, in at least one plate thereof, an outwardly extending channel to accommodate increased width (compared with the thickness of the freezing mixture) of the port and tube.

Some embodiments of the invention will now be described, by way of example only, with reference to the accompanying diagrammatic drawings, of which:
Figure 1 is a plan view of a freezing bag according as described in GB-A-9218538,
Figure 2 is a perspective view of a freezing frame according to the present invention,
Figure 3 is an end elevation of the rear plate of the frame shown in Figure 2, and
Figure 4 is a side elevation, in the direction of the arrow A in Figure 3, of the rear plate.

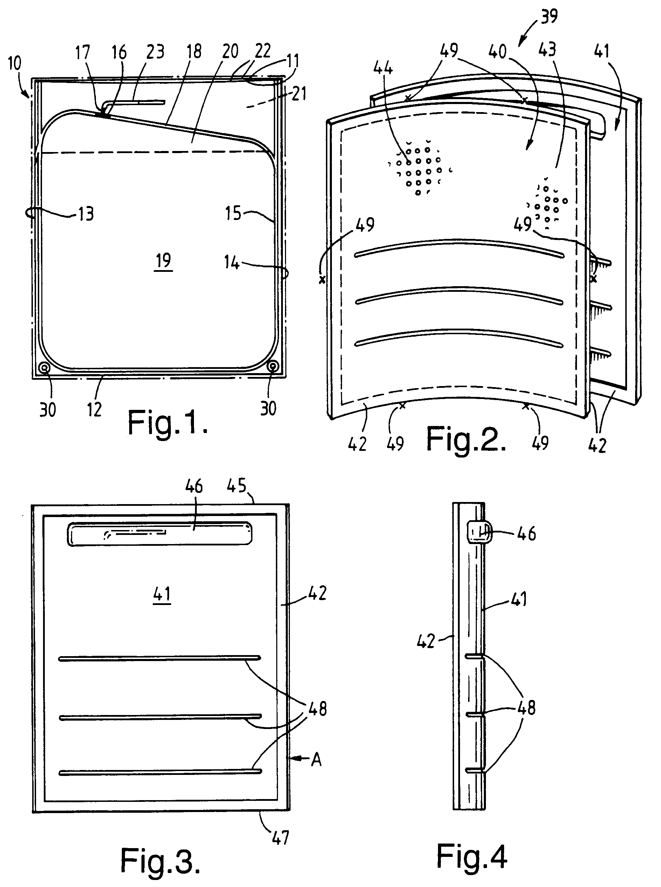

As described in GB-A-9218538 a freezing bag 10 (Figure 1) is formed from two sheets of material, shown generally at 11. A bottom edge 12 and side edges 13, 14 of the sheets are joined together by a first weld 15. An insert 16 having an integral port 17 is positioned towards the top of and between the sheets 11 and the sheets 11 are joined together by a by a second weld 18 which is angled upwardly (relative to a right angle) from the weld 15 at one side 14 of the sheets 11, and which defines an access edge, overlies the insert 16 such that there is a passage across the weld 18, then curves downwardly to meet the weld 15 at the side 14 of the sheets 11. The welds 15, 18 thus define a containment zone 19 which is sealed other than through the insert 16 and port 17, and the insert 16 is positioned towards the top of a pocket 20 defined by the normal to the weld 15 at its junction with the weld 18 and the weld 18.

An open pouch 21 is defined by the extent of the sheets 11 beyond the containment zone 19 and pocket 20, the pouch being defined by the weld 18, extensions of the weld 15, and ends 22 of the sheets 11. A tube 23 is attached to the port 17. In use, after the containment zone 19 has been filled with mixture to be frozen the tube 23 is sealed, air is expelled from the pouch 18 and the ends 22 of the sheets 11 are sealed then usually sealed to contain the tube 23.

In order to meet the stringent requirements of the method of PCT/GB90/0140 (ie controlling an optimum thickness of contents when freezing a standard unit of blood) the containment zone has an area of approximately 0.102 sq. metres, and the welds 15, 18 at the corners of the containment zone 19 are curved. The containment zone 19, apart from the pocket 20, is advantageously rectangular, almost square, in shape, and suitable approximate dimensions are similar to those shown in Figure 1, namely an overall bag length of 395mm and width of 325mm, with lengths between weld 18 and weld 15 at the bottom edge 12, at ends of the pocket 20, being respectively 340 and 300mm with welds 15 18 4mm wide, weld 15 being inset 6mm from edges 14, 13 of sheets 11 and corners having radii of 46mm. At the corners of the bottom edge 12, external to the containment zone 19, are two reinforced hanging apertures 30.

A freezing frame 39 (Figure 2) according to the present invention, in a form suitable for use with the freezing bag described above with reference to Figure 1, has a front plate 40 and a rear plate 41, each of cylindrically curved form with the chord between te edges being displaced about 2cm from the midline. Each plate (see particularly plate 40 in Figure 2) is formed from aluminium and has a solid edging band 42 surrounding a 0.9mm thick grill 43 which has perforations such as those shown at 44. The dimensions are such that when the plates 40, 41 are positioned together with their edging bands 42 in contact there is a displacement of about 3mm between the facing surfaces of the grills 43. The rear plate 41 has (Figures 3, 4) towards a first end 45, a channel 46 protruding outwardly therefrom, and in both plates 40, 41 from a second end 47 extends a series of horizontal ribs 48, of which three are shown in each plate, which protrude inwardly, ribs in the two sheets being staggered.

In use with a bag 10 of the type described above with reference to Figure 1 the frame 39 is positioned to enclose a bag 10, which has been filled with a mixture to be frozen, such that the port 17 and tube 23 of the bag 10 lie within the channel 46. The plates 40, 41 are secured together by six spring clips (not shown) positioned at points 49 (Figure 2). The use of spring clips rather than hinges and clasps, which are prone to icing up during the freezing process, reduces the danger of distortion of the frame 39 when the bag 10 is removed therefrom. The frame 39 and contained bag 10 are then suspended vertically, with second end 47 of the frame 39 lowermost, in liquid nitrogen and the contents frozen in the manner well known in the art. During the freezing process the mixture expands slightly, and the use of spring clips allows the plates 40, 41 to move slightly relative to one another to accommodate this expansion whilst keeping the thickness of the mixture constant. The shape of the plates resists local distortion which would allow perturbations in the thickness, the resistance being increased at the bottom of the frames, where the pressure of the mixture is greater, due to gravity, than at the top, by the ribs 48. The bag 10 will usually be removed from the frame 39 for storage, allowing the frame 39 to be re-used.

When the stored blood is required it is thawed according to the method of PCT/GB/90/00140 and the tube 23 connected by a sterile docking device to any regular transfusion bag. The thawing process, which is extremely simple, is the only task not familiar to those used to giving blood transfusions and the blood for transfusion is presented to medical and nursing staff in a bag with which they are familiar.

It will, of course, be realised that many variations of the above described frame 39 are possible within the scope of the invention. For example, the dimensions quoted are based on the assumption that the frame will be used with a freezing bag 10 as described in GB-A-9218538. Frames according to the invention can, of course, be used with other types of freezing bag, in which case the dimensions may be different and the disposition of the ribs 48 may be other than as shown in Figures 2 to 4.

## Claims

1. A freezing frame having two cylindrically curved perforated plates (40,41) adapted to, in use, contain a freezing bag during a freezing process in which the bag (19) is suspended with its length vertical in a freezing medium, including, on each plate, a series of horizontally disposed ribs (48) extending into the volume of the frame wherein the straight surface lines of the perforated plates extend perpendicularly to said ribs.

2. A freezing frame as claimed in Claim 1 wherein the ribs (48) in the two plates are staggered.

3. A freezing frame as claimed in Claim 1 or in Claim 2 having, in at least one plate thereof, an outwardly extending channel (46).

## Patentansprüche

1. Gefrierrahmen, der zwei zylindrisch gebogene, gelochte Platten (40, 41) aufweist, die so ausgebildet sind, daß sie im Gebrauch während eines Einfriervorgangs einen Gefrierbeutel enthalten können, bei dem der Beutel (19) in der Länge senkrecht in einem Gefriermedium hängt, wobei auf jeder Platte eine Reihe waagerecht angeordneter Rippen (48) vorgesehen sind, die sich in den Innenraum des Rahmens hinein erstrecken, und wobei sich die geraden Mantellinien der gelochten Platten senkrecht zu den Rippen erstrecken.

2. Gefrierrahmen nach Anspruch 1, bei dem die Rippen (48) in den beiden Platten gegenseitig versetzt angeordnet sind.

3. Gefrierrahmen nach Anspruch 1 oder 2, bei dem mindestens eine Platte einen sich nach außen erstreckenden Kanal (46) aufweist.

## Revendications

1. Cadre de congélation comprenant deux plaques perforées à courbure cylindrique (40, 41) qui sont destinées, pendant l'utilisation, à contenir un sachet de congélation au cours d'une opération de congélation dans laquelle le sachet (19) est suspendu avec sa longueur en direction verticale dans un fluide de congélation, le cadre comprenant, sur chaque plaque, une série de crêtes (48) placées horizontalement et pénétrant dans le volume du cadre, les droites génératrices des plaques perforées étant perpendiculaires aux crêtes.

2. Cadre de congélation selon la revendication 1, dans lequel les crêtes (48) des deux plaques sont décalées.

3. Cadre de congélation selon la revendication 1 ou 2, comprenant, dans une plaque au moins, un canal (46) dépassant vers l'extérieur.
